# NEW EUROPEAN PATENT SPECIFICATION

(11) **EP 0 833 662 B2**
(45) Date of publication and mention of the opposition decision: **26.01.2011**
(45) Mention of the grant of the patent: 21.03.2001
(21) Application number: 96922871.7
(22) Date of filing: 19.06.1996
(51) Int. Cl.: A61K 39/39, A61K 39/145

(54) **A vaccine composition comprsing a Haemophilus influenzae B polysaccharide conjugate antigen adsorbed onto aluminium phosphate**
Eine Impfstoffzusammensetzung, bestehend aus einem Haemophilus influenzae B Polysaccharid Antigen-Konjugat adsorbiert an Aluminiumphosphat
Composition vaccinale comprenant un antigène polyosidique conjugué, de hemophilus influenzae B adsorbé sur du phosphate d'aluminium

(30) Priority: 23.06.1995 GB 9512827; 01.07.1995 GB 9513443; 15.12.1995 GB 9525657; 22.03.1996 GB 9606032
(43) Date of publication of application: 08.04.1998
(60) Divisional application: 00203874.3
(73) Proprietor: SmithKline Beecham Biologicals S.A., 1330 Rixensart (BE)
(72) Inventor: PEETERMANS, Julien, B-1330 Rixensart (BE); HAUSER, Pierre, B-1330 Rixensart (BE)
(74) Representative: Dalton, Marcus Jonathan William
(86) International application number: PCT/EP1996/002690
(87) International publication number: WO 1997/000697

(56) References cited:
- EP-A- 0 594 950
- ADV. EXP. MED. BIOL., vol. 303, 1991, IMMUNOBIOLOGY OF PROTEINS AND PEPTIDES VI, ED.: M.Z. ATASSI, PLENUM PRESS, NEW YORK, 1991, pages 185-190, XP000570343 BIXLER, JR., G.S. AND S. PILLAI: "Augmentation by interleukins of the antibody response to a conjugate vaccine against Haemopholus influenza B"
- THE JOURNAL OF PEDIATRICS, vol. 112, 1988, pages 695-702, XP000604106 CLAESSON, B.A. ET AL: "Clinical and immunologic responses to the capsular polysaccharide of Haemophilus influenzae type b alone or conjugated to tetanus toxoid in 18- to 23-month-old children"
- SIBER ET AL. VACCINE vol. 13, no. 6, 1995, pages 525 - 531

## Description

The present invention relates to new vaccine formulations, comprising a conjugated polysaccharide antigen linked to a carrier protein. In particular the invention relates to a vaccine formulation for the prevention of Haemophilus Influenzae Type B (Hib) infections and where the antigen is adsorbed on to aluminium phosphate. The invention also relates to a multivalent vaccine, that is a vaccine for the amelioration or treatment of more than one disease states. The present invention also relates to the production and use of such vaccines in medicine.

Vaccines that utilise polysaccharides are known in the art. For example a vaccine for the prevention of Haemophilus influenzae b (Hib) infections are based on the capsular polysaccharide (PRP) conjugated with a carrier protein. The polysaccharide is a polymer of ribose, ribitol and phosphate. These vaccines are typically presented as plain (ie without adjuvantation) formulations. Although in one case, (Pedvax Hib produce by Merck) a diluent containing aluminium hydroxide is utilised to reconstitute the lyophilised conjugate. Typically the carrier protein is a diphtheria or tetanus toxoid or an outer membrane protein of N. meningitidis. Examples of such conjugate vaccine antigens are disclosed in US 4 365 170, US 4 673 574, EP 208 375, EP 477508 and EP 161 188. Bixler et al. [Adv. Exp. Med. Biol. (1991) 303:185-190] discloses a composition comprising a CRM197 conjugate of Hib polysaccharide adsorbed onto aluminium phosphate, but with no other antigens derived from a pathogen.

It is desirable to administer such conjugate vaccines with other antigens or vaccines at the same time and this can involve multiple injections. Problems associated with multiple injections include a more complicated administration procedure and a large total injection volume. This is a particularly acute problem when the vaccine is intended for infants.

It has therefore been proposed to produce combination vaccines. EP 594950 discloses combination vaccines including unadsorbed Hib polysaccharide conjugates. One well known combination vaccine provides protection against Diphtheria , tetanus and B. pertussis infections. This vaccine comprises a whole cell or an acellular pertussis component which typically consists of two or three antigens - (detoxified PT, FHA and often, but not exclusively 69kDa) although in certain circumstances other B. pertussis antigens may also be present and toxoided diphtheria and tetanus toxins. Such vaccines are often referred to as DTPw or DTPa. Other antigens would desirably be added to such a combination vaccine for the prevention of diseases like hepatitis B or Polio.

It would be desirable to add Hib polysaccharide conjugate vaccines to such a combination. However we have found that simple mixing of the components results in a reduction of antibody titres to the polysaccharide component.

The present inventors have discovered that this reduction can be inhibited if the Hib polysaccharide conjugate antigen is adsorbed on to aluminium phosphate. In contrast, if the antigen is adsorbed on to aluminium hydroxide, there is a complete reduction of antibody titres to the Hib polysaccharide component.

Accordingly the present invention provides a combination as claimed in claim 1.

Preferably the carrier protein is either diphtheria or tetanus toxoid, Diphtheria Crm₁₉₇ protein or an outer membrane protein from a bacteria such as N. meningitidis.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application W093/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide activated polysaccharide derivatised with adipic acid hydrazide (ADH) to the protein carrier by carbodiimide condensation. Such conjugation is described in Chu C. et al Infec Immunity, 1983 245 256.

In a preferred embodiment of the invention the ratio of PRP polysaccharide to carrier protein is reduced from a typical 1:3 to 1:0.3 to 1:2. Such low ratio conjugates are advantageous, since even in an unadjuvanted state, they do not suffer from interference problems.

Preferably, the other antigens in the combination vaccine comprise at least one antigen which affords protection against one or more of the following: Hepatitis A virus (HAV), diphtheria, tetanus, pertussis, Hepatitis B and polio.

Particular combination vaccines within the scope of the invention include a DTPa (diphtheria-tetanus-acellular pertussis) -Hib combination vaccine formulation, a DTPa-Hib-Hepatitis B vaccine formulation and an IPV (inactivated polio vaccine) -DTPa-Hib-Hepatitis B vaccine formulation.

The above combinations may optionally include a component which is protective against Hepatitis A.

Suitable components for use in such vaccines are already commercially available and details may be obtained from the World Health Organisation. For example the IPV component may be the Salk inactivated polio vaccine. The Diphtheria, Tetanus and Pertussis vaccine comprises an acellular product such as Infanrix DTPa (SmithKline Beecham Biologicals) . The component affording protection against Hepatitis A is preferably the product known as 'Havrix' (SmithKline Beecham Biologicals) which is a killed attenuated vaccine derived from the HM-175 strain of HAV [see 'Inactivated Candidate Vaccines for Hepatitis A' by F.E. Andre, A Hepburn and E.D'Hondt, Prog Med. Virol. Vol 37, pages 72-95 (1990) and the product monograph 'Havrix' published by SmithKline Beecham Biologicals (1991)]. The Hepatitis B component may comprise the 'S' antigen as in 'Engerix-B'.

Advantageously the combination vaccine according to the invention is a paediatric vaccine.

Vaccine preparation is generally described in Vaccine Design - The Subunit and adjuvant approach Ed Powell and Newman; Pellum Press. Encapsulation within liposomes is described, for example, by Fullerton, US Patent 4,235,877. Conjugation of proteins to macromolecules is disclosed, for example, by Likhite, US Patent 4,372,945 and by Armor et al., US Patent 4,474,757.

The amount of conjugate antigen in each vaccine dose is selected as an amount which induces an immunoprotective response without significant, adverse side effects in typical vaccinees. Such amount will vary depending on which specific immunogens are employed. Generally it is expected that each dose will comprise 1-1000ug of total immunogen, preferably 2-100ug, most preferably 4-40ug. An optimal amount for a particular vaccine can be ascertained by standard studies involving observation of antibody titres and other responses in subjects. Following an initial vaccination, subjects may receive one or two booster injections at about 4 weeks intervals.

In a further aspect according to the invention, there is provided a method of producing the vaccine comprising adsorbing the Hib polysaccharide conjugate antigen onto aluminium phosphate. The adsorbing is preferably done at a pH of between 5 and 6, preferably at about 5.4. In an embodiment the adsorbed Hib vaccine is freeze dried after standing for more than 24 hours. The adsorbed Hib vaccine of the invention may be combined with the other antigens in a liquid form.

The invention further provides the first medical use of such a vaccine.

In a further embodiment the invention provides a use of a non-toxic and efficacious amount of the combination vaccine of the invention in the manufacture of a medicament for the treatment of a patient suffering from or susceptible to Haemophilus influenzae b infection.

The following examples illustrate the invention.

### Example 1

### Vaccine formulation comprising HiB polysaccharide conjugated on Tetanus toxoid adsorbed on to Aluminium phosphate.

### Synthesis of Haemophilus influenzae type B capsular polysaccharide (PRP) Tetanus toxoid (TT) conjugate

### 1.a Cyanogen Bromide Coupling

The covalent binding of PRP and TT is carried out by a coupling chemistry developed at the NIH (Chu C. et al (1983), further studies on the immunogenicity of Haemophilus influenzae type b and pneumococcal type 6A polysaccharide protein conjugates. Infec. Immunity, 245-256). The PRP is activated under controlled conditions by cyanogen bromide and derivatised with an adipic hydrazide spacer.

After derivatisation, the activated polysaccharide (PRP-AH) is purified by diafiltration. The coupling of the two purified components (PRP-AH and TT) is effected by carbodiimide condensation. The conjugate is then purified by ultrafiltration and gel filtration to remove the reagent and unconjugated PRP and TT.

### Synthesis of PRP-TT Conjugates

### 1.b CDAP coupling

30mg of native Hib PRP were dissolved in 6ml 2M NaCl. 225 µl (mcl) of CDAP (1 cyano-4-dimethylamino-pyridinum tetrafluoroborate) was added to the polysaccharide solution (from a 100 mg/ml stock solution in acetonitrile). 90 seconds later, 450 µl (mcl) of 0.2 M triethylamine was added. The activation was performed at pH 10.0 during 1 minute on ice and minute at room temperature.

90 mg of tetanus toxoid (initial PS/protein ratio of 1/3) were added to the activated polysaccharide and the coupling reaction was performed at room temperature for 1 hour. Then, the reaction was quenched with 3 ml of 1M glycine solution, pH 5.0 for 30 minutes at room temperature and overnight at 4°C.

The conjugate was purified by gel filtration on a sephacryl HR 500 column equilibrated in 0.2M NaCl. The carbohydrate and protein content was determined in each fraction. The conjugate was pooled and sterile filtered (membrane Minisart ⊕ 0.222 µm).

### Adsorption on to aluminium phosphate

1.c To 0.15mg of aluminium phosphate was added 12.5 µg (mcg) of the polysaccharide conjugate of example 1(a). This was stirred for two hours the pH is adjusted to 5.1. The mixture was left to stand for one day at room temperature and the adsorbed conjugate then left for a further 9 days at 2 to 8 °C. To prepare a freeze dried product the adsorbed product is diluted in lactose (15.75mg) to give a final composition of 25 µg (mcg) polysaccharide/ml and 0.4mg Al/ml and the resulting composition was filled into 0.5ml vials and freezed dried.

To prepare a liquid product the adsorbed conjugate is diluted in water for injection with 150mM NaCl and 5mg/ml phenoxy ethanol to give a final composition of 20 µg (mcg) polysaccharide/ml and 0.32 mg Al/ml.

1.d Formulation of a Diphtheria Tetanus and Pertussis (acellular) vaccine with and without hepatitis B was done in accordance to the methods of WO 93/24148 (SmithKline Beecham Biologicals).

### 1.e Preparation of a 'low ratio' PRP-TT aluminium phosphate pre-adsorbed conjugate.

The conjugate was prepared in an analogous manner to the example of 1a, but with reduced amount of Tetanus used [30 µg (mcg), 60 µg (mcg)] to give a product with Polysaccharide:Protein ratio of 1:1 or 1:2. The conjugate is then adsorbed on to aluminium phosphate according to the method of example 1c. The final freeze dried preparation contains 12.5µg of conjugate, 0.15mg AIPO₄, 15.75 mg lactose. This is reconstituted in 0.5ml water for injection prior to use at a pH of 0.1 +/- 0.1.

### Example 2: Immunogenicity of PRP-TT conjugate preadsorbed on aluminium phosphate and combined with DTPa or DTPa-HB

The Hib conjugate of example 1a), either plain or pre-adsorbed on AIPO₄ (both vaccines were lyophilized) was mixed with DTPa or DTPa HB no more than 1 hour before injection and the combination was injected in baby rats (1 week of age) by the subcutaneous route at a dose corresponding to 1/20th a human dose (0.5 µg of PRP). The rats were boosted 2 weeks and 4 weeks later and the serum was collected was collected after each immunization to measure anti-PRP antibodies. Controls included the Hib vaccines (adsorbed or not on AIPO₄) reconstituted in saline.

Groups of 10 randomized baby rats (1 week of age-OFA strain) were immunized 3 times subcutaneously at 0-14-28 days with 1/20th human dose of Hib vaccine, alone or combined with DTPa or DTPa HB (1/20th a human dose). The reconstitution of the lyophilised Hib vaccine with saline or combinations (DTPa or DTPa HB) was done less than 1 hour before immunization.

The rats were bled under anaesthesia at 14-28-42 and 56 days. The anti-PRP antibodies were measured by ELISA in individual sera and the titers were expressed in µg/ml (γ/ml) using a calibrated reference. The GMT was calculated for each group and for each time point. The 95% confidence limits were calculated for the titers obtained after that third immunization.

As shown in table 1, the adsorption of Hib conjugate on AIPO₄ does not modify its immunogenicity: some anti-PS were produced after the second dose and a good booster effect is shown after the third dose as seen in human babies. The mixing of Hib vaccine with DTPa or DTPa HB reduces by 3 to 8 fold the anti-PRP response and, in the case of DTPa-HB, this decrease is significant. In contrast, the pre-adsorption of the Hib vaccine on AIPO₄ restores the anti-PRP response to a level at least equivalent to that obtained with the plain vaccine.

### Conclusion:

The Hib/aluminium phosphate formulation has thus the potential to solve the compatibility problem encountered when mixing Hib with other peadiatric combinations.

**Table 1**

| Immunogenicity in a baby rat model of PRP-TT conjugate pre adsorbed on AIPO₄ and combined with DTPa or DTPa-HB | | | | |
|---|---|---|---|---|
| **Vaccine** | **Anti-PRP titre (µg/ml [γ/ml]) at day** | | | |
| | **14 (Post I)** | **28 (Post II)** | **42 (Post III)** | **56 (Post III 30)** |
| None (Nacl 0.9%) | <0.05 | <0.05 | <0.05 | <0.05 |
| Hib-001 | <0.05 | 0.06 | 12.9 (4-37) | 10.9 (4-31) |
| Hib/AIP04 (Dhib-024) | <0.05 | 1.3 | 11.8 (5-29) | 15.4 (7-35) |
| Hib-001+DTPa (119) | <0.05 | 0.16 | 3.4 (0-28) | 1.4 (0.1-17) |
| Hib/AIP04 + DTPa (119) | <0.05 | 1.9 | 20.9 (7-59) | 19.7 (9.42) |
| Hib-001+DTPa HB (16705) | <0.05 | 0.14 | 2.8 (1-6) | 3.9 (2-9) |
| Hib/AIP04 + DTPa HB (16705) | <0.05 | 0.47 | 11.4 (5-27) | 18.1 (9-38) |
| Hib/Al(OH₃) | <0.05 | <0.05 | <0.05 | <0.14 |

## Claims

1. A combination vaccine comprising:
i) a capsular polysaccharide of Haemophilus influenzae B conjugated to a carrier protein **characterised in that** the conjugate is adsorbed onto aluminium phosphate; and
ii) other antigens which afford protection against diphtheria, tetanus and pertussis disease.

2. A combination vaccine as claimed in claim 1 wherein the conjugate is admixed with one or more other antigens which afford protection against a disease selected from the group: Hepatitis A, Hepatitis B and Polio.

3. A combination vaccine as claimed in claim 1 or 2 wherein the carrier protein is selected from the group comprising: Diphtheria toxoid, Diphtheria CRM197 protein and Meningococcal outer membrane protein.

4. A combination vaccine as claimed in claim 1 or 2 wherein the carrier protein conjugated to the capsular polysaccharide of Haemophilus influenzae B is tetanus toxoid.

5. A combination vaccine as claimed in any one of claims 1 to 4 wherein the ratio of the Haemophilus influenzae B polysaccharide to carrier protein is from 1:0.3 to 1:2 (w:w).

6. A combination vaccine as claimed in any one of claims 1 to 5 wherein the adsorbed conjugate has been freeze dried prior to its combination with the other antigens.

7. A kit for making a combination vaccine comprising a container of a freeze-dried vaccine comprising a capsular polysaccharide of Haemophilus influenzae B conjugated to a carrier protein and adsorbed onto aluminium phosphate, and a second container with a vaccine which affords protection against diphtheria, tetanus and pertussis disease.

8. A method of producing a vaccine according to claims 1 to 6 comprising conjugating a Haemophilus influenzae type B capsular polysaccharide antigen to a protein carrier, adsorbing said conjugate onto aluminium phosphate, and mixing with said other antigens.

9. The method of claim 8 wherein said other antigens afford protection against a disease selected from the group consisting of: Hepatitis A, diphtheria, tetanus, pertussis, Hepatitis B and Polio.

10. A vaccine composition as defined in any of the claims 1 to 6 for use in medicine.

11. The use of a safe and efficacious amount of a vaccine composition as defined in any of claims 1 to 6 in the manufacture of a medicament for the treatment of a patient suffering from or susceptible to Haemophilus influenzae B infection.

12. The combination vaccine of claim 2 wherein the capsular polysaccharide of Haemophilus influenzae B is conjugated to tetanus toxoid, and wherein the vaccine comprises the following antigens: diphtheria toxoid, tetanus toxoid, acellular pertussis antigens, Hepatitis B surface antigen and Inactivated Polio Vaccine.

## Patentansprüche

1. Kombinationsimpfstoff, umfassend:
(i) ein Kapselpolysaccharid von Haemophilus influenzae B, konjugiert an ein Trägerprotein, **dadurch gekennzeichnet, dass** das Konjugat an Aluminiumphosphat adsorbiert ist; und
(ii) weitere Antigene, die Schutz gegen Diphtherie-, Tetanus- und Pertussis-Erkrankung gewähren.

2. Kombinationsimpfstoff nach Anspruch 1, worin dem Konjugat ein oder mehrere weitere Antigene, die Schutz gegen eine Erkrankung, ausgewählt aus der Gruppe: Hepatitis A, Hepatitis B und Polio, gewähren, beigemischt sind.

3. Kombinationsimpfstoff nach Anspruch 1 oder 2, worin das Trägerprotein ausgewählt ist aus der Gruppe, umfassend: Diphtherietoxoid, Diphtherie CRM197-Protein und äusseres Meningokokken-Membranprotein.

4. Kombinationsimpfstoff nach Anspruch 1 oder 2, worin das Trägerprotein, das an das Kapselpolysaccharid von Haemophilus influenzae B konjugiert ist, Tetanustoxoid ist.

5. Kombinationsimpfstoff nach einem der Ansprüche 1 bis 4, worin das Verhältnis von Haemophilus influenzae B-Polysaccharid zu Trägerprotein bei 1:0,3 bis 1:2 (G:G) liegt.

6. Kombinationsimpfstoff nach einem der Ansprüche 1 bis 5, worin das adsorbierte Konjugat vor seiner Zumischung zu den weiteren Antigenen gefriergetrocknet worden ist.

7. Kit zur Herstellung eines Kombinationsimpfstoffs, umfassend einen Behälter eines gefriergetrockneten Impfstoffs, umfassend ein Kapselpolysaccharid von Haemophilus influenzae B, das an ein Trägerprotein konjugiert und an Aluminiumphosphat adsorbiert ist, und einen zweiten Behälter mit einem Impfstoff, der Schutz gegen Diphtherie-, Tetanus- und Pertussis-Erkrankung gewährt.

8. Verfahren zur Herstellung eines Impfstoffs nach den Ansprüchen 1 bis 6, umfassend das Konjugieren eines Haemophilus influenzae Typ B-Kapselpolysaccharid-Antigens an ein Trägerprotein, Adsorbieren des Konjugats an Aluminiumphosphat und Mischen mit den weiteren Antigenen.

9. Verfahren nach Anspruch 8, wobei die weiteren Antigene Schutz gegen eine Erkrankung, ausgewählt aus der Gruppe bestehend aus: Hepatitis A, Diphtherie, Tetanus, Pertussis, Hepatitis B und Polio, gewähren.

10. Impfstoffzusammensetzung nach einem der Ansprüche 1 bis 6 zur Verwendung in der Medizin.

11. Verwendung einer sicheren und wirksamen Menge einer Impfstoffzusammensetzung nach einem der Ansprüche 1 bis 6 bei der Herstellung eines Medikaments zur Behandlung eines Patienten, der an einer Haemophilus influenzae B-Infektion leidet oder für sie empfänglich ist.

12. Kombinationsimpfstoff nach Anspruch 2, worin das Kapselpolysaccharid von Haemophilus influenzae B an Tetanustoxoid konjugiert ist und worin der Impfstoff folgende Antigene umfasst: Diphtherietoxoid, Tetanustoxoid, azelluläre Pertussis-Antigene, Hepatitis B-Oberflächenantigen und inaktivierter Polioimpfstoff.

## Revendications

1. Vaccin mixte comprenant :
i) un polysaccharide capsulaire de *Haemophilus influenza* B conjugué à une protéine porteuse, **caractérisé en ce que** le produit conjugué est adsorbé sur du phosphate d'aluminium ; et
ii) d'autres antigènes qui offrent une protection contre la diphtérie, le tétanos et la coqueluche.

2. Vaccin mixte selon la revendication 1, dans lequel le produit conjugué est mélangé avec un ou plusieurs autres antigènes qui offrent une protection contre une maladie sélectionnée dans le groupe suivant : hépatite A, hépatite B et polio.

3. Vaccin mixte selon la revendication 1 ou 2, dans lequel la protéine porteuse est sélectionnée dans le groupe comprenant : une anatoxine diphtérique, une protéine diphtérique CRM197 et une protéine de la membrane externe des méningocoques.

4. Vaccin mixte selon la revendication 1 ou 2, dans lequel la protéine porteuse conjuguée au polysaccharide capsulaire de *Haemophilus influenza* B est une anatoxine tétanique.

5. Vaccin mixte selon l'une quelconque des revendications 1 à 4, dans lequel le rapport du polysaccharide de *Haemophilus influenza* B sur la protéine porteuse est de 1/0,3 à 1/2 (p/p).

6. Vaccin mixte selon l'une quelconque des revendications 1 à 5, dans lequel le produit conjugué adsorbé a été cryodesséché avant sa combinaison avec les autres antigènes.

7. Kit permettant de fabriquer un vaccin mixte comprenant un récipient de vaccin cryodesséché comprenant un polysaccharide capsulaire de *Haemophilus influenza* B conjugué à une protéine porteuse et adsorbé sur du phosphate d'aluminium, et un second récipient contenant un vaccin qui offre une protection contre la diphtérie, le tétanos et la coqueluche.

8. Procédé de production d'un vaccin selon les revendications 1 à 6, comprenant la conjugaison d'un antigène de polysaccharide capsulaire de *Haemophilus influenza* B à une protéine porteuse, l'adsorption dudit produit conjugué sur du phosphate d'aluminium et le mélange avec lesdits autres antigènes.

9. Procédé selon la revendication 8, dans lequel lesdits autres antigènes offrent une protection contre une maladie sélectionnée dans le groupe suivant : hépatite A, diphtérie, tétanos, coqueluche, hépatite B et polio.

10. Composition vaccinale selon l'une quelconque des revendications 1 à 6 pour un usage médical.

11. Utilisation d'une quantité sûre et efficace d'une composition vaccinale selon l'une quelconque des revendications 1 à 6, dans la fabrication d'un médicament destiné au traitement d'un patient souffrant d'une, ou sensible à une, infection à *Haemophilus influenza* B.

12. Vaccin mixte selon la revendication 2, dans lequel le polysaccharide capsulaire de *Haemophilus influenza* B est conjugué à une anatoxine tétanique, et où le vaccin comprend les antigènes suivants : une anatoxine diphtérique, une anatoxine tétanique, des antigènes pertussiques acellulaires, un antigène de surface de l'hépatite B et un vaccin inactivé contre la polio.
